(19) Europäisches Patentamt
European Patent Office
Office européen des brevets

(11) Veröffentlichungsnummer: **0 221 445 B1**

(12) **EUROPÄISCHE PATENTSCHRIFT**

(45) Veröffentlichungstag der Patentschrift: **26.10.88**

(51) Int. Cl.⁴: **C 07 D 321/06**, A 61 K 7/46

(21) Anmeldenummer: **86114626.4**

(22) Anmeldetag: **22.10.86**

(54) **Substituierte 5-Formyl-1,3-dioxepane, deren Herstellung und Verwendung als Riechstoffe.**

(30) Priorität: **07.11.85 DE 3539468**

(43) Veröffentlichungstag der Anmeldung: **13.05.87 Patentblatt 87/20**

(45) Bekanntmachung des Hinweises auf die Patenterteilung: **26.10.88 Patentblatt 88/43**

(84) Benannte Vertragsstaaten: **CH DE ES FR GB IT LI NL**

(56) Entgegenhaltungen:
**EP - A - 0 085 937**
**DE - B - 2 407 863**

(73) Patentinhaber: **BASF Aktiengesellschaft, Carl-Bosch-Strasse 38, D-6700 Ludwigshafen (DE)**

(72) Erfinder: **Gramlich, Walter, Dr., Auf der Höhe 11, D-6803 Edingen-Neckarhausen (DE)**
Erfinder: **Siegel, Hardo, Dr., Hans-Purrmann-Allee 25, D-6720 Speyer (DE)**
Erfinder: **Jahn, Dieter, Dr., Burgunder Weg 8, D-6803 Edingen-Neckarhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 221 445 B1

**Beschreibung**

Die Erfindung betrifft substituierte 5-Formyl-1,3-dioxepane der allgemeinen Formel I

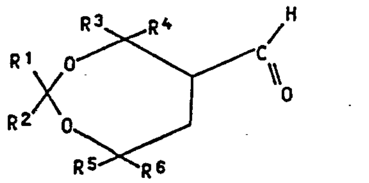

(I),

in der $R^1$ für einen geradkettigen oder verzweigten Alkylrest mit 1 bis 10 C-Atomen, der auch Sauerstoff in Form einer Ethergruppierung enthalten kann oder einen gegebenenfalls durch Alkylgruppen und/oder eine Alkylengruppe substituierten 5- bis 8-gliedrigen Ring mit bis zu 10 C-Atomen, der auch Sauerstoff in Form einer Ethergruppierung enthalten kann, vorzugsweise für einen geradkettigen oder verzweigten Alkylrest mit 1 bis 8 C-Atomen und $R^2$ für Wasserstoff steht,
oder aber $R^1$ und $R^2$ zusammen für einen gegebenenfalls durch Alkyl- oder Alkoxygruppen substituierten Alkylenrest mit 4 bis 10 C-Atomen, der auch Sauerstoff in Form einer Ethergruppierung enthalten kann, vorzugsweise für einen Alkylenrest mit 4 bis 6 C-Atomen, stehen
und $R^3$, $R^4$, $R^5$ und $R^6$ Wasserstoff oder Methyl bedeuten und
in der die Gesamt-C-Atomzahl maximal 25 beträgt.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an Geruchsverbesserern für Produkte des täglichen Bedarfs, wie Reinigungsmittel, Kosmetika, Leime etc. hat die Riechstoffindustrie ständig Bedarf an neuen Riechstoffen, die allein oder in Form von Kompositionen wertvolle Parfums bzw. Duftstoffe mit interessanten Duftnoten darstellen. Da aufgrund wenig bekannter Zusammenhänge zwischen Struktur und Riechstoffeigenschaften eine gezielte Synthese von Riechstoffen mit gewünschten olfaktorischen Eigenschaften nicht möglich ist, besteht die Aufgabe, solche Verbindungen aufzufinden, die wertvolle Riechstoffqualitäten besitzen.

Es wurde nun zufällig gefunden, dass die oben beschriebenen Verbindungen der Formel I eine neue Verbindungsklasse mit hochinteressanten Geruchsnoten darstellen.

Die Erfindung betrifft daher auch die Verwendung der substituierten 5-Formyl-1,3-dioxepane der allgemeinen Formel I als Riechstoffe sowie diese Dioxepane, vorzugsweise in einer Menge von 1 bis 50 Gew.-%, bezogen auf die ganze Komposition, enthaltende Riechstoffkompositionen.

Gegenstand der Erfindung ist weiterhin ein Verfahren zur Herstellung der Verbindungen der allgemeinen Formel I, das dadurch gekennzeichnet ist, dass man

A. eine Carbonylverbindung der allgemeinen Formel II

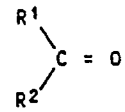

(II),

in der $R^1$ und $R^2$ die in Anspruch 1 angegebene Bedeutung haben, oder deren Acetale bzw. Ketale mit Alkendiolen der allgemeinen Formel III

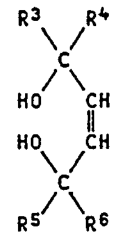

(III),

in der $R^3$ bis $R^6$ die in Anspruch 1 angegebene Bedeutung haben, in Gegenwart eines sauren Katalysators cyclisiert, und

B. die dabei erhaltenen substituierten 4,7-Dihydro-1,3-dioxepine der allgemeinen Formel IV

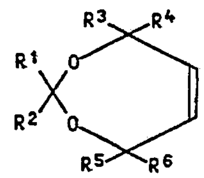

(IV)

in an sich bekannter Weise hydroformyliert.

Als Carbonylverbindung der Formel II kommen dabei Formaldehyd ($R^1=R^2=$Wasserstoff), aliphatische Aldehyde ($R^1=$Wasserstoff, $R^2=$Alkylreste) oder cycloaliphatische Ketone in Betracht.

Als Aldehyde seien beispielsweise genannt:

Acetaldehyd, Propionaldehyd, 2-Methyl-propanal, n-Butyraldehyd, 2-Methylbutanal, 3-Methyl-butanal, 3-Ethyl-butanal, Valeraldehyd, 2-Methyl-pentanal, 3-Methyl-pentanal, 4-Methylpentanal, 2,2-Dimethyl-propanal (Pivalinaldehyd) sowie unsubstituierte höhere Aldehyde wie n-Hexanal, n-Heptanal, n-Octanal, n-Nonanal, Decanal sowie deren ein- und/oder mehrfach alkylierte Derivate. Bekannteste Vertreter sind das chemische Grossprodukt 2-Ethyl-hexanal sowie 3,5,5-Trimethyl-hexanal.

Als Sauerstoff-enthaltende Carbonylkomponenten seien beispielsweise genannt:

Methoxyacetaldehyd, 3-Isobutoxy-propanal, Methoxyaceton und Methoxybutanon.

Als Carbonylverbindungen, die 5- bis 8-gliedrige, gegebenenfalls Sauerstoff-haltige Ringe mit bis zu 10 C-Atomen enthalten, seien beispielsweise genannt:

Cyclohexan-carbaldehyd, Tetrahydropyran-2-carbaldehyd, Tetrahydropyran-3-carbaldehyd und 2,7,7-Trimethyl-bicyclo[3.1.1$^{1.5}$]heptan-3-carbaldehyd.

Als cyclische Ketone, bei denen die Reste $R^1$ und $R^2$ Teile eines 5- bis 8-gliedrigen, gegebenenfalls Sauerstoff enthaltenden Ringes mit bis zu 10 C-Atomen bedeuten, sind beispielsweise geeignet:

Cyclopentanon (5 C-Atome), 2,4,4-Trimethylcyclopentanon (8 C-Atome), Cyclohexanon,

2,3,6-Trimethyl-cyclohexanon (9 C-Atome), Cycloheptanon und Cyclooctanon.

Als Alkendiole der allgemeinen Formel III können beispielsweise eingesetzt werden:

2-Buten-1,4-diol, 2-Penten-1,4-diol, 3-Hexen-2,5-diol, 4-Methyl-2-penten-1,4-diol, 2-Methyl-3-hexen-2,5-diol und 2,5-Dimethyl-3-hexen-2,5-diol.

Die aufgeführten Alkendiole lassen sich leicht z. B. durch partielle Hydrierung der entsprechenden Alkindiole mit Lindlar-Katalysatoren herstellen bzw. stellen selbst technische Gross- und Zwischenprodukte dar, wie das 2-Buten-1,4-diol, das aus Acetylen und zwei Molekülen Formaldehyd und Partialhydrierung des dabei gebildeten 2-Butin-1,4-diols erhältlich ist. 2,5-Dimethyl-3-hexin-2,5-diol lässt sich leicht durch Umsetzung von Acetylen und Aceton herstellen (vgl. DAS 1816042).

Die gut zugänglichen Carbonylverbindungen der Formel II werden im Reaktionsschritt A mit den Alkendiolen der Formel III in Gegenwart üblicher saurer Katalysatoren wie p-Toluolsulfonsäure und meist eines Schleppmittels, wie Toluol oder Cyclohexan, unter Wasserauskreisen cyclisiert. Eine Acetalisierung der Carbonylverbindungen mit niederen Alkoholen vor der Cyclisierung erwies sich in den meisten Fällen als nicht vorteilhafter als die Cyclisierung der Carbonylverbindungen selbst.

Die bei der Cyclisierung erhaltenen alkylierten zum grossen Teil neuen 4,7-Dihydro-1,3-dioxepine der Formel IV werden ausschliesslich zu den 5-Formyl-1,3-dioxepanen der Formel I hydroformyliert.

Die Hydroformylierung wird in an sich bekannter Weise (vgl. z. B. J. Falbe, Carbon Monoxide in Organic Synthesis, Springer-Verlag 1970, insbesondere S. 35) durchgeführt.

Als Katalysatoren für die Hydroformylierungsreaktion eignet sich vor allem Rhodium als fein verteiltes Metall, Oxid oder als Salz einer organischen oder anorganischen Säure, gegebenenfalls in Gegenwart von tertiären Phosphinen oder Phosphiten in einem bis zu 300fachen Überschuss bezogen auf Rhodium. Geeignet sind auch Rhodiumcarbonylverbindungen oder Rhodiumkomplexe, welche man durch Umsetzung von Rhodiumsalzen oder Rhodiumcarbonylverbindungen mit Phosphin oder Phosphiten und Olefinen oder mehrfach ungesättigten Olefinen erhält. Auch bei diesen Komplexen können gegebenenfalls Phosphine und Phosphite bis zum 300fachen Überschuss, bezogen auf Rhodium, zugegen sein.

Die Katalysatoren können in homogener oder heterogener Form (beispielsweise auf Trägern) eingesetzt werden.

Die Katalysatorkonzentration liegt bei 1 bis 1000 ppm Rhodium bezogen auf eingesetztes Substrat.

Kohlenmonoxid und Wasserstoff werden vorzugsweise in etwa äquimolaren Mengen eingesetzt, jedoch ist es möglich, von jedem der Gase einen bis zu etwa 4fachen molaren Überschuss zu verwenden.

Vorzugsweise nimmt man die Hydroformylierung bei 70 bis 150° C und 10 bis 700 bar Kohlenmonoxid/Wasserstoff Gesamtdruck, gegebenenfalls in einem inerten Lösungs- oder Verdünnungsmittel wie Benzol, Toluol, Xylolgemischen, Leichtbenzin, Ethern oder Estern vor.

Sie lässt sich kontinuierlich oder diskontinuierlich durchführen.

Die Aufarbeitung der Reaktionsgemische auf die Verfahrensprodukte kann in an sich bekannter Weise, z. B. durch Destillation, erfolgen.

Die erhaltenen, alkylierten 5-Formyl-1,3-dioxepane der Formel I stellen eine hochinteressante neue Klasse von Riechstoffen mit unterschiedlichen, charakteristischen neuen Geruchsnoten dar.

So weist z. B. die Verbindung 2-tert.-Butyl-5-formyl-1,3-dioxepan, die aus Pivalaldehyd und 2-Buten-1,4-diol und anschliessende Hydroformylierung leicht zugänglich ist, eine hochinteressante Geruchskombination auf, die sowohl an die Frische des Meeres als auch an Maiglöckchen erinnert.

2-Isopropyl-5-formyl-1,3-dioxepan weist nur eine Methyl-Gruppe weniger als die tert.-Butyl-Verbindung auf, besitzt jedoch einen hiervon völlig verschiedenen, grün-holzigen Geruch.

Die 2-n-Propyl-Verbindung als Homologes besitzt hingegen eine feine süsswürzige Estragol-Note.

Setzt man n-Hexanal mit 2-Buten-1,4-diol um und hydroformyliert das Cyclisierungsprodukt, so gelangt man zu 5-Formyl-2-pentyl-1,3-dioxepan, das eine hochinteressante krautig-grüne Note aufweist, die an Rhabarber erinnert.

Die in Tabellenform dargestellte Übersicht der olfaktorischen Eigenschaften der wichtigsten erfindungsgemässen Verbindungen verdeutlicht die grosse Vielfalt und Bedeutung der neuen Verbindungen und zeigt darüber hinaus, welch überraschend grosse Geruchsveränderungen durch kleine Veränderungen am Molekül hervorgerufen werden.

Wenn $R^1$ und $R^2$ verschiedene Bedeutungen haben, dann liegen die erfindungsgemässen Verbindungen als E/Z-Diastereomerengemische (meist 1:1) vor. Eine destillative Anreicherung eines Diastereomeren ist möglich.

Die erfindungsgemässen Verbindungen können daher in stereoisomeren Formen sowohl als Diastereomere als auch als Enantiomere vorliegen.

Wesentlich für die olfaktorischen Eigenschaften ist die Stellung der Formylgruppe im Dioxepanring. Sonst gleiche Dioxepane, die die Formylgruppe anstatt in 5-Stellung in 4- oder 7-Stellung tragen, zeigten in allen untersuchten Fällen sehr schlechte olfaktorische Eigenschaften, so dass sie als Riechstoffe keine Bedeutung besitzen.

Die erfindungsgemässen substituierten 5-Formyl-1,3-dioxepane lassen sich gut mit anderen Riechstoffen in verschiedenen unterschiedlichen Mengenverhältnissen zu neuartigen, interessanten Riechstoffkompositionen kombinieren.

Ausser in der Feinparfümerie können derartige Kompositionen zur Parfümierung von Kosmetika wie Cremes, Lotionen, Aerosolen, Toilettenseifen, technischen Artikeln wie Reinigungs- und

Waschmitteln, Weichspülern, Desinfektionsmitteln und Textilbehandlungsmitteln dienen.

Von ganz besonderer Bedeutung als Riechstoffe für die Feinparfümerie sind substituierte 5-Formyl-1,3-dioxepane der allgemeinen Formel I, in der $R^1$ für einen geradkettigen oder verzweigten Alkylrest mit 1 bis 8 C-Atomen steht. $R^2$ für Wasserstoff steht oder aber $R^1$ und $R^2$ zusammen für einen Alkylenrest mit 4 bis 6 C-Atomen stehen und $R^3$, $R^4$, $R^5$ und $R^6$ Wasserstoff oder Methyl bedeuten. Genannt seien insbesondere

2-Methyl-5-formyl-1,3-dioxepan,
2-tert.-Butyl-5-formyl-1,3-dioxepan,
2-Isopropyl-5-formyl-1,3-dioxepan,
2-Pentyl-5-formyl-1,3-dioxepan,
2-Pentyl-4,7-dimethyl-5-formyl-1,3-dioxepan,
2-Propyl-5-formyl-1,3-dioxepan,
2-(2,4,4-Trimethyl-pentyl)-5-formyl-1,3-dioxepan,
9-Formyl-7,12-dioxaspiro[5.6]dodecan,
2,4,7-Trimethyl-5-formyl-1,3-dioxepan, und
2,4,4,7,7-Pentamethyl-5-formyl-1,3-dioxepan.

*Beispiel 1:*

Herstellung von 2-tert.-Butyl-5-formyl-1,3-dioxepan

A. Ein Gemisch aus 430 g (5 mol) 2,2-Dimethyl-propanal, 3 g p-Toluolsulfonsäure, 440 g (5 mol) 2-Buten-1,4-diol in 1000 ml Toluol wurde unter Rückfluss zum Sieden erhitzt und innerhalb von 3 Stunden (h) das sich bildende Reaktionswasser entfernt. Anschliessend liess man auf Raumtemperatur (RT) abkühlen, versetzte die Lösung mit 10 ml einer 25%igen Natronlauge, wusch einmal mit wenig Wasser neutral und destillierte das Toluol unter vermindertem Druck bei 50 bis 60° C ab. Der erhaltene Rückstand wurde bei 0,1 mbar destilliert. Man erhielt 702 g 2-tert.-Butyl-4,7-dihydro-1,3-dioxepin. Kp. 80° C/32 mbar.

B1. In einem 1-l-Magnethubrührautoklaven wurden 430 g eines gemäss A. hergestellten 2-tert.-Butyl-4,7-dihydro-1,3-dioxepins, 300 ml Toluol und 52 mg $(Rh(COD)Cl)_2$ (COD=Cyclooctadienyl-1,5) vorgelegt. Anschliessend presste man kalt 300 bar $CO/H_2$ (1:1) auf, erwärmte auf 90° C und hielt während 12 h einen Druck von 650 bar $CO/H_2$ aufrecht. Nach Abkühlen und Entspannen arbeitete man das Reaktionsgemisch destillativ (Kurzweg) auf. Man erhielt 456 g 2-tert.-Butyl-5-formyl-1,3-dioxepan vom Kp.=122° C/35 mbar. Das Produkt enthielt ca. 10% des 4-Formyl-Isomeren, welches sich leicht destillativ abtrennen liess.

B2. In einem 300-ml-Rührautoklaven wurden 50 g 2-tert.-Butyl-4,7-dihydro-1,3-dioxepin (hergestellt gemäss A.), 100 ml Toluol und 100 ppm Rh als $(Rh(COD)Cl)_2$ (COD=Cyclooctadienyl-1,5) sowie 1 g Triphenylphosphin vorgelegt. Man presste kalt 30 bar $CO/H_2$ (1:1) auf, erwärmte auf 90° C und hielt während 12 h einen Druck von 200 bar aufrecht. Nach destillativer Aufarbeitung analog Beispiel 1 B1. erhielt man 52 g 5-Formyl-2-tert.-butyl-1,3-dioxepan, Kp. 124° C/35 mbar. Das entsprechende 4-Formyl-Isomere liess sich gaschromatographisch nicht nachweisen.

Analog Beispiel 1 wurden die in Tabelle 1 aufgeführten Verbindungen hergestellt.

*Tabellen am Ende des Patents*

*Beispiel 11:*

Herstellung von 2-Propyl-4,7-dimethyl-5-formyl-1,3-dioxepan

A. Ein Gemisch aus 580 g (5 mol) 3-Hexen-2,5-diol, 3 g p-Toluolsulfonsäure, 360 g (5 mol) n-Butanal und 1 l Toluol wurde unter Rückfluss zum Sieden erhitzt und innerhalb von 3 h das anfallende Reaktionswasser (108 ml) entfernt. Anschliessend liess man auf RT abkühlen, versetzte mit 10 ml einer 25%igen wässerigen Natronlauge, wusch mit wenig Wasser neutral und entfernte das Toluol unter vermindertem Druck bei 50 bis 60° C. Der erhaltene Rückstand wurde bei 0,1 mbar destilliert. Man erhielt 681 g (4 mol) 2-Propyl-4,7-dimethyl-4,7-dihydro-1,3-dioxepin. Kp. 70° C/0,1 mbar.

B. In einem 1-l-Magnethubrührautoklaven wurden 400 g 2-Propyl-4,7-dimethyl-4,7-dihydro-1,3-dioxepin (hergestellt gemäss A), 300 ml Toluol und 52 mg $(Rh(COD)Cl)_2$ (COD=Cyclooctadienyl-1,5) vorgelegt. Anschliessend presste man kalt 300 bar $CO/H_2$ (1:1) auf, erwärmte auf 90° C und hielt während 12 h einen Druck von 650 bar $CO/H_2$ aufrecht. Nach Abkühlen und Entspannen wurde das Reaktionsgemisch destillativ in einer Kurzwegdestillationsapparatur aufgearbeitet. Man erhielt 410 g 2-Propyl-4,7-dimethyl-5-formyl-1,3-dioxepan.

Analog Beispiel 11 bzw. analog Beispiel 11A und Beispiel 1B2 wurden die in Tabelle 2 aufgeführten Verbindungen hergestellt.

*Tabellen am Ende des Patents*

*Beispiel 16:*

Herstellung von 2-Pentyl-4,4,7,7-tetramethyl-5-formyl-1,3-dioxepan

A. Ein Gemisch aus 720 g (5 mol) 2,5-Dimethyl-3-hexen-2,5-diol, 3 g p-Toluolsulfonsäure, 500 g (5 mol) n-Hexanal und 1 l Toluol wurde unter Rückfluss zum Sieden erhitzt und innerhalb von 5,5 h das angefallene Reaktionswasser (115 ml) abgetrennt. Anschliessend liess man abkühlen, versetzte mit 10 ml einer 25%igen wässerigen Natronlauge, wusch einmal mit wenig Wasser neutral und entfernte das Toluol unter vermindertem Druck bei 50 bis 60° C. Der erhaltene Rückstand wurde bei 0,1 mbar destilliert. Man erhielt 796 g 2-Pentyl-4,4,7,7-tetramethyl-4,7-dihydro-1,3-dioxepin vom Kp.=75° C/0,1 mbar.

B. In einem 300-ml-Rührautoklaven wurden 40 g 2-Pentyl-4,4,7,7-tetramethyl-4,7-dihydro-1,3-dioxepin, 100 ml Toluol und 104 mg $(Rh(COD)Cl)_2$ sowie 1 g Triphenylphosphin vorgelegt. Man presste kalt 30 bar $CO/H_2$ (1:1) auf, erwärmte auf 90° C und hielt innerhalb von 12 h einen Druck von 200 bar aufrecht. Nach destillativer Aufarbeitung erhielt man 41 g 2-Pentyl-5-formyl-4,4,7,7-tetramethyl-1,3-dioxepan vom Kp.=130° C/0,005 mbar.

Analog Beispiel 16 bzw. analog Beispiel 16A und 1 B1 wurden die in Tabelle 3 aufgeführten Verbindungen hergestellt.

*Tabellen am Ende des Patents*

*Anwendungsbeispiel*

Die bedeutenden Einsatzmöglichkeiten der neuen Verbindungen soll anhand der Wirkung von 2-tert.-Butyl-5-formyl-1,3-dioxepan auf eine bekannte Blumen-Base angedeutet werden.

Beispiel A: Klassische Muguet-Base Nr. 1 (aus E.V. Wells, M. Billot, Perfumery Technology, John Wiley & Sons, 2nd Ed. 1981, S. 223).

| | A Gew.-Teile | B Gew.-Teile |
|---|---|---|
| Citronellol | 33,7 | 33,7 |
| Rhodinol P | 11,3 | 11,3 |
| Phenylethanol | 24,7 | 24,7 |
| Hydroxycitronellal | 14,6 | 10,0 |
| Benzoesäurebenzylester | 6,7 | 6,7 |
| Phthalsäurediethylester | 4,5 | 4,5 |
| α-Amyl-zimtaldehyd | 2,25 | 2,25 |
| Indol 10% | 2,25 | 2,25 |
| 2-tert.-Butyl-5-formyl-1,3-dioxepan | — | 4,6 |
| | 100 | 100 |

Durch Substitution von 4,6-Teilen Hydroxycitronellal durch 2-tert.-Butyl-5-formyl-1,3-dioxepan wirkt die Muguet-Base (B) stärker, abgerundeter und noch erheblich frischer.

**Patentansprüche**

1. Substituierte 5-Formyl-1,3-dioxepane der allgemeinen Formel I

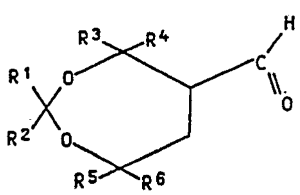

(I),

in der $R^1$ für einen geradkettigen oder verzweigten Alkylrest mit 1 bis 10 C-Atomen, der auch Sauerstoff in Form einer Ethergruppierung enthalten kann, oder einen gegebenenfalls durch Alkylgruppen und/oder eine Alkylengruppe substituierten 5- bis 8-gliedrigen Ring mit bis zu 10 C-Atomen, der auch Sauerstoff in Form einer Ethergruppierung enthalten kann, steht und $R^2$ Wasserstoff bedeutet
oder aber $R^1$ und $R^2$ zusammen für einen gegebenenfalls durch Alkyl- oder Alkoxygruppen substituierten Alkylenrest mit 4 bis 10 C-Atomen, der auch Sauerstoff in Form einer Ethergruppierung enthalten kann, stehen
und $R^3$, $R^4$, $R^5$ und $R^6$ Wasserstoff oder Methyl bedeuten und
in der die Gesamt-C-Atomzahl maximal 25 beträgt.

2. Substituierte 5-Formyl-1,3-dioxepane der allgemeinen Formel I

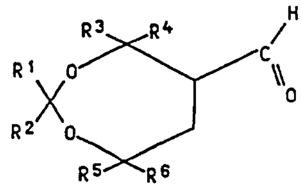

(I),

in der $R^1$ für einen geradkettigen oder verzweigten Alkylrest mit 1 bis 8 C-Atomen und $R^2$ für Wasserstoff steht,
oder aber $R^1$ und $R^2$ zusammen für einen Alkylenrest mit 4 bis 6 C-Atomen stehen
und $R^3$, $R^4$, $R^5$ und $R^6$ Wasserstoff oder Methyl bedeuten und in der die Gesamt-C-Atomzahl maximal 25 beträgt.

3. 2-Methyl-5-formyl-1,3-dioxepan.

4. 2-tert.-Butyl-5-formyl-1,3-dioxepan.

5. 2-Isopropyl-5-formyl-1,3-dioxepan.

6. 2-Pentyl-5-formyl-1,3-dioxepan.

7. 2-Propyl-5-formyl-1,3-dioxepan.

8. 9-Formyl-7,12-dioxa-spiro[5.6]dodecan.

9. 2-(2,4,4-Trimethyl-pentyl)-5-formyl-1,3-dioxepan.

10. 2-Pentyl-4,7-dimethyl-5-formyl-1,3-dioxepan.

11. 2,4,7-Trimethyl-5-formyl-1,3-dioxepan.

12. 2,4,4,7,7-Pentamethyl-5-formyl-1,3-dioxepan.

13. Verwendung von substituierten 5-Formyl-1,3-dioxepanen der allgemeinen Formel I gemäss Anspruch 1, als Riechstoffe.

14. Riechstoffkompositionen, *gekennzeichnet durch* einen Gehalt an substituierten 5-Formyl-1,3-dioxepanen der Formel I gemäss Anspruch 1.

15. Riechstoffkompositionen nach Anspruch 4, *dadurch gekennzeichnet*, dass sie die 5-Formyl-1,3-dioxepane in einer Menge von 1 bis 50 Gew.%, bezogen auf die gesamte Komposition, enthalten.

16. Verfahren zur Herstellung von Verbindungen der allgemeinen Formel I gemäss Anspruch 1, *dadurch gekennzeichnet*, dass man

A. eine Carbonylverbindung der allgemeinen Formel II

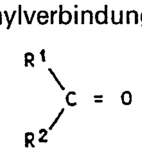

(II),

in der $R^1$ und $R^2$ die in Anspruch 1 angegebene Bedeutung haben, oder deren Acetale bzw. Ketale mit Alkendiolen der allgemeinen Formel III

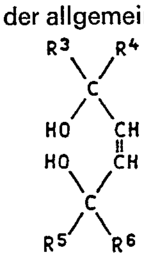

(III),

in der $R^3$ bis $R^6$ die in Anspruch 1 angegebene Bedeutung haben, in Gegenwart eines sauren Katalysators cyclisiert, und

B. die dabei erhaltenen substituierten 4,7-Dihydro-1,3-dioxepine der allgemeinen Formel IV

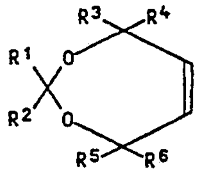

(IV)

in an sich bekannter Weise hydroformyliert.

## Claims

1. A substituted 5-formyl-1,3-dioxepane of the formula

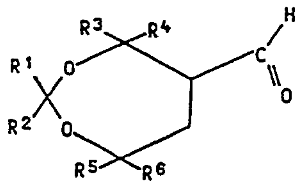

(I)

where $R^1$ is a straight-chain or branched alkyl radical of 1 to 10 carbon atoms which may furthermore contain oxygen in the form of an ether group, or a 5-membered to 8-membered ring which is unsubstituted or substituted by alkyl groups and/or an alkylene group, has not more than 10 carbon atoms and may furthermore contain oxygen in the form of an ether group, $R^2$ is hydrogen, or $R^1$ and $R^2$ together form an alkylene radical of 4 to 10 carbon atoms which is unsubstituted or substituted by alkyl or alkoxy and may furthermore contain oxygen in the form of an ether group, and $R^3$, $R^4$, $R^5$ and $R^6$ are each hydrogen or methyl, and the total number of carbon atoms is not more than 25.

2. A substituted 5-formyl-1,3-dioxepane of the formula

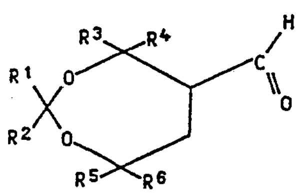

(I)

where $R^1$ is a straight-chain or branched alkyl radical of 1 to 8 carbon atoms, $R^2$ is hydrogen, or $R^1$ and $R^2$ together form an alkylene radical of 4 to 6 carbon atoms, and $R^3$, $R^4$, $R^5$ and $R^6$ are each hydrogen or methyl, and the total number of carbon atoms is not more than 25.

3. 2-methyl-5-formyl-1,3-dioxepane.

4. 2-tert-butyl-5-formyl-1,3-dioxepane.

5. 2-isopropyl-5-formyl-1,3-dioxepane.

6. 2-pentyl-5-formyl-1,3-dioxepane.

7. 2-propyl-5-formyl-1,3-dioxepane.

8. 9-formyl-7,12-dioxa-spiro[5.6]dodecane.

9. 2-(2,4,4-trimethyl-pentyl)-5-formyl-1,3-dioxepane.

10. 2-pentyl-4,7-dimethyl-5-formyl-1,3-dioxepane.

11. 2,4,7-trimethyl-5-formyl-1,3-dioxepane.

12. 2,4,4,7,7-pentamethyl-5-formyl-1,3-dioxepane.

13. Use of a substituted 5-formyl-1,3-dioxepane of the formula I as claimed in claim 1 as a scent.

14. A scent composition which contains a substituted 5-formyl-1,3-dioxepane of the formula I as claimed in claim 1.

15. A scent composition as claimed in claim 4, which contains a 5-formyl-1,3-dioxepane in an amount of from 1 to 50% by weight, based on the total composition.

16. A process for the preparation of a compound of the formula I as claimed in claim 1, wherein

A. a carbonyl compound of the formula II

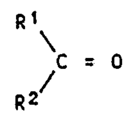

(II)

where $R^1$ and $R^2$ have the meanings stated in claim 1, or one of its acetals or ketals is subjected to a cyclization reaction with an alkenediol of the formula III

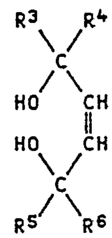

(III)

where $R^3$, $R^4$, $R^5$ and $R^6$ have the meanings stated in claim 1, in the presence of an acidic catalyst, and

B. the resulting substituted 4,7-dihydro-1,3-dioxepin of the formula IV

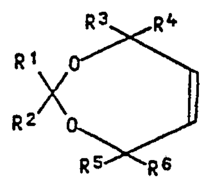

(IV)

is hydroformylated in a conventional manner.

## Revendications

1. 5-formyl-1,3-dioxépannes substitués de formule générale I

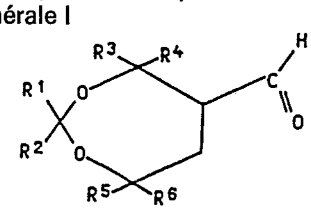

(I)

dans laquelle $R^1$ représente un radical alkyle à chaîne droite ou ramifiée contenant 1 à 10 atomes de carbone et pouvant aussi contenir de l'oxygène

sous la forme d'un groupement éther, ou un cycle à 5 à 8 chaînons contenant jusqu'à 10 atomes de carbone, éventuellement substitué par des groupes alkyle et/ou un groupe alkylène et pouvant aussi contenir de l'oxygène sous forme d'un groupement éther, et $R^2$ représente de l'hydrogène, ou bien

$R^1$ et $R^2$ représentent ensemble un radical alkylène à 4 à 10 atomes de carbone, éventuellement substitué par des groupes alkyle ou alcoxy et pouvant aussi contenir de l'oxygène sous forme d'un groupement éther, et

$R^3$, $R^4$, $R^5$ et $R^6$ représentent de l'hydrogène ou des radicaux méthyle,
et dans laquelle le nombre total d'atomes de carbone est au maximum de 25.

2. 5-formyl-1,3-dioxépannes substitués de formule générale I

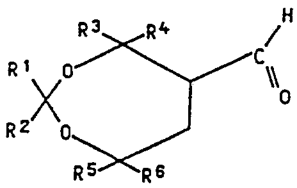

(I)

dans laquelle $R^1$ représente un radical alkyle à chaîne droite ou ramifiée contenant 1 à 8 atomes de carbone et $R^2$ représente de l'hydrogène, ou bien

$R^1$ et $R^2$ représentent ensemble un radical alkylène contenant 4 à 6 atomes de carbone, et

$R^3$, $R^4$, $R^5$ et $R^6$ représentent de l'hydrogène ou des radicaux méthyle,
et dans laquelle le nombre total d'atomes de carbone est au maximum de 25.

3. 2-méthyl-5-formyl-1,3-dioxépanne.

4. 2-tert.-butyl-5-formyl-1,3-dioxépanne.

5. 2-isopropyl-5-formyl-1,3-dioxépanne.

6. 2-pentyl-5-formyl-1,3-dioxépanne.

7. 2-propyl-5-formyl-1,3-dioxépanne.

8. 9-formyl-7,12-dioxa-spiro[5.6]dodécane.

9. 2-(2,4,4-triméthyl-pentyl)-5-formyl-1,3-dioxépanne.

10. 2-pentyl-4,7-diméthyl-5-formyl-1,3-dioxépanne.

11. 2,4,7-triméthyl-5-formyl-1,3-dioxépanne.

12. 2,4,4,7,7-pentaméthyl-5-formyl-1,3-dioxépanne.

13. Utilisation de 5-formyl-1,3-dioxépannes substitués de la formule générale I suivant la revendication 1, comme matières odorantes.

14. Compositions renfermant des matières odorantes, caractérisées en ce qu'elles contiennent des 5-formyl-1,3-dioxépannes substitués de la formule I suivant la revendication 1.

15. Compositions renfermant des matières odorantes suivant la revendication 14, caractérisées en ce qu'elles contiennent les 5-formyl-1,3-dioxépannes en une quantité de 1 à 50% en poids par rapport à la composition totale.

16. Procédé de préparation de composés de la formule générale I suivant la revendication 1, caractérisé en ce que

A. on cyclise en présence d'un catalyseur acide un composé carbonylé de formule générale II

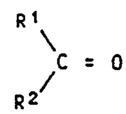

(II)

dans laquelle $R^1$ et $R^2$ ont les significations indiquées dans la revendication 1, ou les acétals ou cétals correspondants avec des alcène-diols de formule générale III

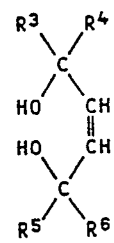

(III)

dans laquelle les radicaux $R^3$ à $R^6$ ont les significations indiquées dans la revendication 1, et

B. on hydroformyle de façon connue en soi les 4,7-dihydro-1,3-dioxépines substituées ainsi obtenues de formule générale IV

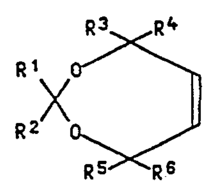

(IV)

*Tabelle 1*

| Bei-spiel | Carbonyl-verbindung | -4,7-dihydro-1,3-dioxepin | Kp (° C/mbar) | -5-formyl-1,3-dioxepan | Kp (° C/mbar) | $n_D^{25}$ | Geruchs-beschreibung |
|---|---|---|---|---|---|---|---|
| 1 | 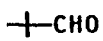 | 2-tert.-Butyl- | 80/32 | 2-tert.-Butyl- | 125/34<br>67/05 | 1,4569 | frische Meeresbrise Maiglöckchen |
| 2 | 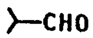 | 2-Isopropyl- | 75/32 | 2-Isopropyl- | 122/35<br>60/0,4 | 1,4517 | grün holzig |
| 3 | $CH_3$-CHO | 2-Methyl- | 145/ND | 2-Methyl- | 66/0,005 | 1,4523 | grün fruchtig |
| 4 | 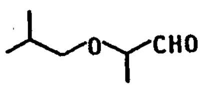 | 2-[1'-Isobutoxy-ethyl]- | 71/0,3 | 2-[1'-Isobutoxy-ethyl]- | 100/2 | 1,4520 | sehr frisch Ozon-artig |
| 5 | 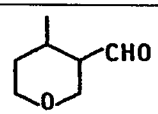 | 2-[2,3,4,5,6-Penta-hydro-4-methyl-pyran-3-yl]- | 95/0,3 | 2-[2,3,4,5,6-Penta-hydro-4-methyl-pyran-3-yl]- | 145/0,05 | 1,830 | blumig |
| 6 | 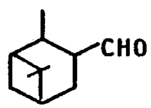 | 2-(2,7,7-Trimethyl-bicyclo[3.1.1.$^{1.5}$]-heptan-3-yl)- | 120/0,3 | 2-(2,7,7-Trimethyl-bicyclo[3.1.1.$^{1.5}$]-heptan-3-yl)- | 115/0,5 | 1,4929 | grün, krautig |
| 7 | 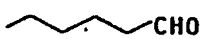 | 2-Pentyl- | 78/30 | 2-Pentyl- | 100/0,01 | 1,4558 | krautig grün Rhabarber-Note |
| 8 | 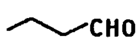 | 2-Propyl- | 107/94 | 2-Propyl- | 65/0,2 | 1,4531 | süss-würzig, Estragol-artig |
| 9 | 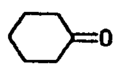 | 7,12-Dioxa-spiro-[5.6]dodeca-9-en- | 105/5 | 9-Formyl-7,12-dioxa-spiro-[5.6]dodecan- | 92/0,01 | 1,4832 | balsamisch, süsslich, fruchtig |
| 10 | 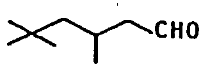 | 2-[2,4,4-Trimethyl-pentyl]- | 123/6 | 2-[2,4,4-Trimethyl-pentyl]- | 120/0,2 | 1,4562 | würzig, krautig |

*Tabelle 2*

| Beispiel | Carbonylverbindung | -4,7-dimethyl-4,7-dihydro-1,3-dioxepin | Kp (° C/mbar) | -4,7-dimethyl-5-formyl-1,3-dioxepan | Kp (° C/mbar) | $n_D^{25}$ | Geruchsbeschreibung |
|---|---|---|---|---|---|---|---|
| 11 | 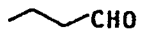 | 2-Propyl- | 60/0,1 | 2-Propyl- | 63/0,1 | 1,4459 | Grün-Note, Bohnen-artig |
| 12 | 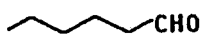 | 2-Pentyl- | 74/0,1 | 2-Pentyl- | 94/0,6 | 1,4487 | frisch, Aldehyd-artig |
| 13 | $CH_3CHO$ | 2-Methyl- | 50/0,1 | 2-Methyl- | 55/0,3 | 1,4552 | Calmus-artig, würzig |
| 14 | 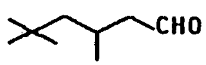 | 2-[2,4,4-Trimethyl-pentyl]- | 90/0,01 | 2-[2,4,4-Trimethyl-pentyl]- | 99/0,001 | 1,4518 | Ozon-artig, frisch |
| 15 | 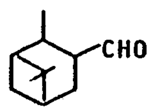 | 2-(2,7,7-Trimethyl-bicyclo-[3.1.1$^{1.5}$]-heptan-3-yl)- | 76/0,01 | 2-(2,7,7-Trimethyl-bicyclo-[3.1.1$^{1.5}$]-heptan-3-yl)- | 117/0,001 | 1,4840 | krautig, Sellerie-artig |

*Tabelle 3*

| Beispiel | Carbonylverbindung | -4,4,7,7-tetramethyl-1,3-dioxepin | Kp (° C/mbar) | -4,4,7,7-tetramethyl-5-formyl-1,3-dioxepan | Kp (° C/mbar) | $n_D^{25}$ | Geruchsbeschreibung |
|---|---|---|---|---|---|---|---|
| 16 | 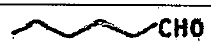 | 2-Pentyl- | 75/0,1 | 2-Pentyl- | 98/0,005 | 1,4552 | krautig, grün |
| 17 | 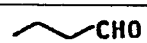 | 2-Propyl- | 60/0,1 | 2-Propyl- | 86/0,15 | 1,4535 | Holz-Note |
| 18 | $CH_3-CHO$ | 2-Methyl- | 50/0,1 | 2-Methyl- | Fp. 78° C | — | Eichenmoos-artig |
| 19 | 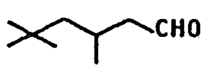 | 2-[2,4,4-Trimethyl-pentyl]- | 81/0,1 | 2-[2,4,4-Trimethyl-pentyl]- | 124/0,015 Fp. 49° C | — | Grün-Note Bohnen-artig |
| 20 | 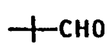 | 2-tert.-Butyl- | 74/18 | 2-tert.-Butyl- | 68/0,005 | 1,4551 | frische Aldehyd-Note |
| 21 | 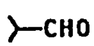 | 2-Isopropyl- | 74/13 | 2-Isopropyl- | 82/0,01 Fp. 44° C | — | süsslich, fruchtig |
| 22 | 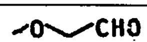 | 2-Methoxymethyl- | 46/0,015 | 2-Methoxymethyl- | 98/0,035 | 1,4580 | blumig, frisch |